# EUROPÄISCHE PATENTANMELDUNG

(11) **EP 1 325 919 A1**
(43) Veröffentlichungstag der Anmeldung: **09.07.2003**
(21) Anmeldenummer: 02027958.4
(22) Anmeldetag: 13.12.2002
(51) Int. Cl.: C07D 319/06, C07D 317/26, C07D 317/20, C07D 317/30

(54) **Verfahren zur Herstellung von Polyendialdehydmonoacetalen**

(30) Priorität: 04.01.2002 DE 10200130
(71) Anmelder: BASF AKTIENGESELLSCHAFT, 67056 Ludwigshafen (DE)
(72) Erfinder: Ernst, Hansgeorg, 67346 Speyer (DE); Henrich, Klaus, 67454 Hassloch (DE)
(74) Vertreter: Thalhammer, Wolfgang, Dr.

(57) **Zusammenfassung**

Beschrieben wird ein Verfahren zur Herstellung von Verbindungen der Formel I 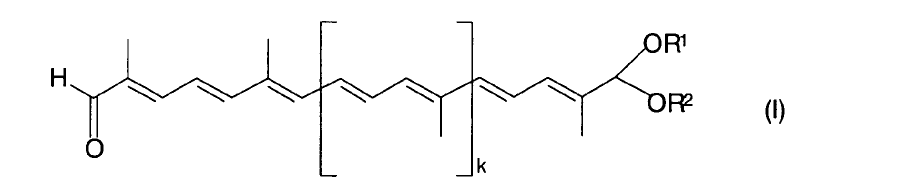 bei dem man eine Verbindung der Formel II 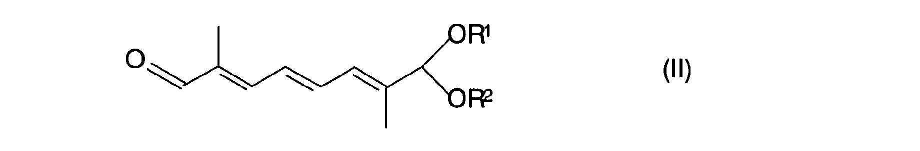 durch Wittig- bzw. Wittig-Horner-Reaktion in eine Verbindung der Formel IV umwandelt, 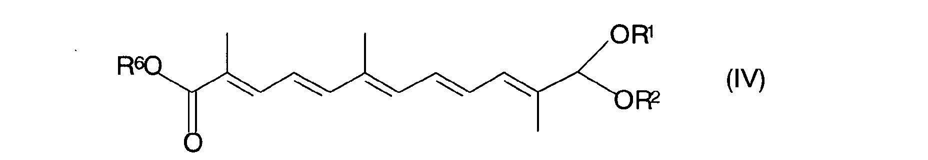 gegebenenfalls die Verbindung der Formel IV durch Hydrolyse der Acetalfunktion und Wittig- bzw. Wittig-Horner-Reaktion in eine Verbindung der Formel VI umwandelt 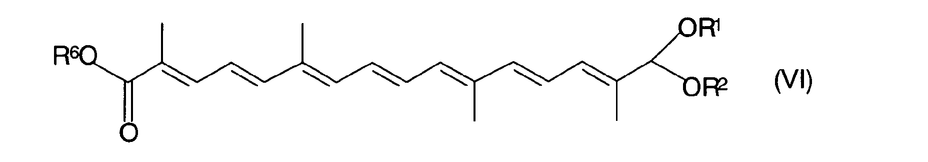 und die Verbindung der Formel IV oder VI in zwei Stufen in die Verbindung der Formel I umwandelt. Die Reste R¹, R² und R⁶ und k haben die in der Beschreibung angegebene Bedeutung. Beschrieben werden auch neue Zwischenprodukte.

## Beschreibung

Die vorliegende Erfindung betrifft ein Verfahren zur Herstellung von C₁₅- bzw. C₂₀-Polyendialdehydmonoacetalen.

Derartige einseitig geschützte Polyenbausteine sind für die selektive Synthese verschiedener unsymmetrisch aufgebauter C₄₀-Carotinoide durch sukzessive Umsetzung des C₁₅-Dialdehydbausteins mit entsprechend funktionalisierten C₁₅- bzw. C₁₀-Bausteinen oder des C₂₀-Dialdehydbausteins mit entsprechend funktionalisierten unterschiedlichen C₁₀-Bausteinen von großem Interesse. Zu den unsymmetrisch aufgebauten C₄₀-Carotinoiden gehören beispielsweise Capsanthin und Cryptocapsin sowie α-Carotin oder β-Cryptoxanthin.

Die DE-A 2851051 beschreibt die Herstellung von 11-(5,5-Dimethyl-1,3-dioxan-2-yl)-2,4,6,8,10-dodecapentaenal durch Wittigoder Wittig-Horner-Olefinierung von 2,6-Dimethylocta-2,4,6-trien-1,8-dial mit einem geeigneten C₅-Baustein unter speziellen Reaktionsbedingungen. Nachteilig an diesem Verfahren ist jedoch, dass der eingesetzte unsymmetrische C₁₀-Dialdehyd kein im technischen Maßstab übliches Synthon ist.

Synth. Met. 42, 1557 (1991) beschreibt die Herstellung von Crocetindial-mononeopentylglycolacetal durch partielle Hydrolyse von Crocetindial-bisneopentylglycolacetal. Das Monoacetal wird chromatographisch gereinigt. Üblicherweise ist die Selektivität einer partiellen Acetalspaltung unzureichend, was das beschriebene Verfahren für einen industriellen Prozess nicht wünschenswert erscheinen lässt.

J. Chem. Soc., Chem. Commun. 1977, 467 und J. Chem. Soc. Perkin Trans 1 (1988) 1383 beschreiben die Herstellung von 11-(1,3-Dioxolan-2-yl)-2,6-dimethyl-2,4,6,8,10-dodecapentaensäuremethylester durch Acetalisierung des Aldehyds mit Ethylenglykol.

Der Erfindung liegt die Aufgabe zugrunde, ein im technischen Maßstab realisierbares und hinsichtlich der Schutzgruppe flexibles Verfahren zur Herstellung von C₁₅- bzw. C₂₀-Dialdehydmonoacetalen bereit zu stellen.

Erfindungsgemäß wird diese Aufgabe gelöst durch ein Verfahren zur Herstellung von Verbindungen der Formel I 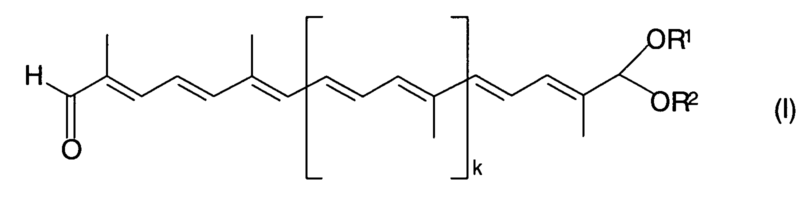 bei dem man
a) eine Verbindung der Formel II 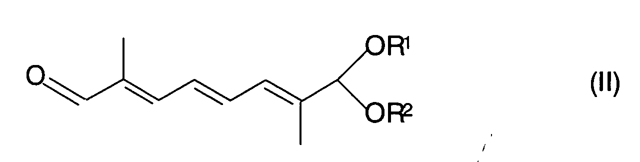 durch Umsetzung mit einem Reagenz der Formel III 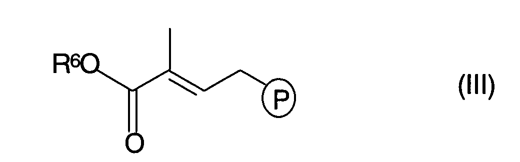 in eine Verbindung der Formel IV umwandelt, 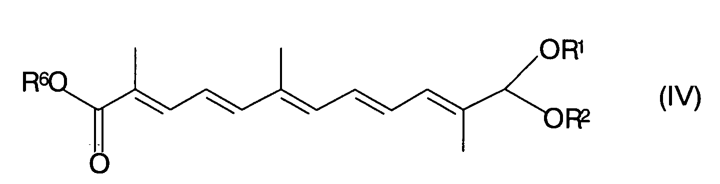
b) gegebenenfalls die Verbindung der Formel IV durch Hydrolyse der Acetalfunktion und Umsetzung mit einem Reagenz der Formel V 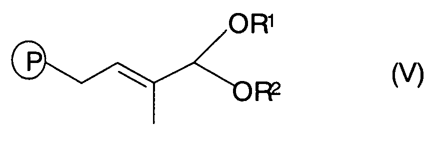 in eine Verbindung der Formel VI umwandelt 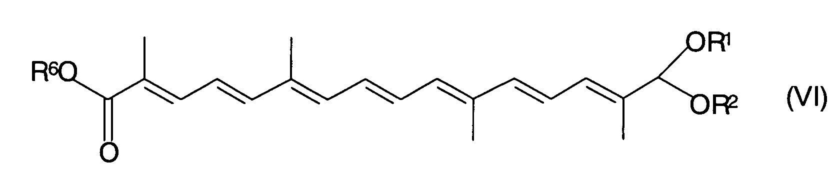
c) die Verbindung der Formel IV oder VI zu einer Verbindung der Formel VII reduziert 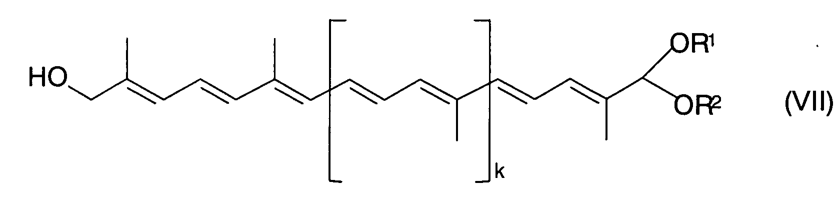
d) die Verbindung der Formel VII zur Verbindung der Formel I oxidiert,
   worin R¹ und R² unabhängig voneinander für C₁-C₈-Alkyl stehen oder gemeinsam mit den Sauerstoffatomen, an die sie gebunden sind, und dem dazwischen liegenden Kohlenstoffatom für 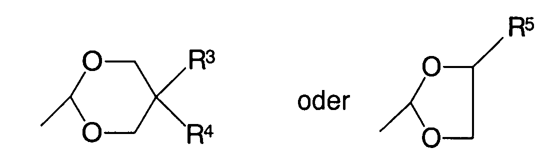 stehen, worin R³, R⁴ und R⁵ unabhängig voneinander für Wasserstoff oder C₁-C₄-Alkyl stehen,
   R⁶ für C₁-C₈-Alkyl steht,
   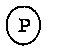 für einen Triarylphosphonium- oder Phosphonsäuredialkylesterrest steht, und
   k für 0 oder 1 steht.

Im Falle von offenkettigen Acetalen seien als Alkylreste für R¹ und R² lineare oder verzweigte C₁-C₈-Alkylketten, z. B. Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl und n-Octyl genannt. Bevorzugte Alkylreste für R¹ und R² sind Methyl, Ethyl, n-Propyl und 1-Methylethyl, besonders bevorzugt Methyl und Ethyl.

Als Alkylreste für R³ bis R⁵ seien lineare oder verzweigte C₁-C₄-Alkylketten, z. B. Methyl, Ethyl, n-Propyl, 1-Methylethyl, n-Butyl, 1-Methylpropyl, 2-Methylpropyl und 1,1-Dimethylethyl genannt. Bevorzugte Reste für R³ bis R⁵ sind Wa'sserstoff und Methyl.

Verbindungen der Formel II sind bekannt und beispielsweise durch p-Toluolsulfonsäure-katalysierte Acetalisierung von 2,7-Dimethylocta-2,4,6-trien-1,8-dial zugänglich, vergleiche *Helv. Chim. Acta* 1981, 64 (7), 2469. Ein bevorzugtes Verfahren zur Herstellung der Verbindungen der Formel II ist in der älteren Anmeldung DE 101 12 067.2 beschrieben. Dabei wird ein 3-Methyl-2-buten-1,4-dial-monoacetal mit einem Reagenz der obigen Formel III umgesetzt, der erhaltene Acetal-ester zum Alkohol reduziert und der Alkohol zu einer Verbindung der Formel II oxidiert.

Vorzugsweise verwendet man eine Verbindung der Formel IIa, 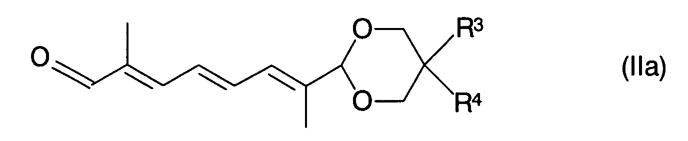 worin R³ und R⁴ gleich sind und für Wasserstoff oder Methyl stehen.

In Schritt a) des erfindungsgemäßen Verfahrens wird die Verbindung der Formel II mit einem Reagenz der Formel III in einer Wittig- oder Wittig-Horner-Reaktion umgesetzt.

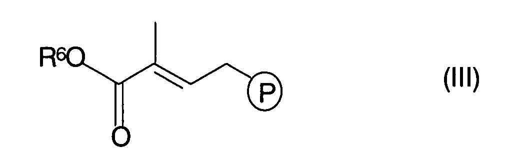

In der Formel III kann (P) für einen Triarylphosphoniumrest P(R⁸)₃+ stehen, wobei R⁸ für übliche, in Phosphinen und Phosphoniumsalzen vorkommende Arylreste, wie Phenyl, Tolyl, Naphthyl, die gegebenenfalls substituiert sein können, steht. Bevorzugt steht R⁸ für Phenyl. Die positive Ladung des Triarylphosphoniumrestes wird durch ein Anionenäquivalent X⁻ einer anorganischen oder organischen Säure, bevorzugt einer starken anorganischen oder organischen Säure, kompensiert.

Der Ausdruck starke Säure umfasst Halogenwasserstoffsäuren, insbesondere Salzsäure und Bromwasserstoffsäure, Schwefelsäure, Phosphorsäure, Sulfonsäuren und andere anorganische oder organische Säuren mit vergleichbarem Dissoziationsgrad. Als starke organische Säuren sind in diesem Zusammenhang auch C₁-C₆-Alkansäuren wie Ameisensäure, Essigsäure, Propionsäure, Buttersäure sowie Capronsäure zu verstehen.

Besonders bevorzugt sind die Anionen von Salzsäure, Bromwasserstoffsäure, Schwefelsäure, Phosphorsäure, Ameisensäure, Essigsäure und/oder Sulfonsäuren. Ganz besonders bevorzugt sind Cl-, Br- und die Anionen von Alkansulfonsäuren mit 1 bis 4 Kohlenstoffatomen, Benzolsulfonsäure, p-Toluolsulfonsäure oder Trifluormethansulfonsäure.

Alternativ steht (P) für einen Phosphonsäuredialkylesterrest PO(OR⁷)₂, worin R⁷ für C₁-C₈-Alkyl steht. In einer bevorzugten Ausführungsform des erfindungsgemäßen Verfahrens setzt man in Schritt a) ein Reagenz der Formel IIIa ein 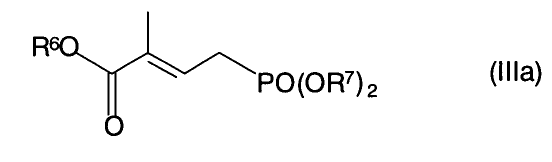 worin R⁶ die bereits angegebene Bedeutung hat und R⁷ für C₁-C₃-Alkyl steht.

Die Umsetzung zwischen der Verbindung der Formel II und dem Reagenz der Formel III wird unter Bedingungen durchgeführt, die für eine Wittig- bzw. Wittig-Horner-Reaktion typisch sind, vgl. z. B. Carotenoids, Vol. 2 "Synthesis", S. 79 ff.; Birkhäuser Verlag, 1996, und die dort zitierte Literatur.

Die Kondensation von II mit einer Verbindung der Formel III, worin (P) für einen Triarylphosphoniumrest steht, kann beispielsweise in einem inerten organischen Lösungsmittel z. B. in offenkettigen oder cyclischen Ethern wie Diethylether, Diisopropylether, Methyl-tert.-butylether, 1,4-Dioxan oder Tetrahydrofuran, in halogenierten Kohlenwasserstoffen wie Dichlormethan, Chloroform, in aromatischen Kohlenwasserstoffen wie Toluol, Xylol oder Benzol oder in polaren Lösungsmitteln wie Dimethylformamid, Dimethylsulfoxid oder Acetonitril durchgeführt werden. Bevorzugte Lösungsmittel sind Toluol, Tetrahydrofuran und Dimethylsulfoxid oder Mischungen davon.

Als Base können alle für Wittig-Kondensationen üblichen Basen verwendet werden, z. B. Alkalimetallhydroxide wie Natriumhydroxid, Kaliumhydroxid oder Lithiumhydroxid; Alkalimetallhydride wie Natriumhydrid oder Kaliumhydrid; oder Alkalimetallalkoholate wie Natriummethylat oder Natriumethylat. Als Basen kommen außerdem Lithiumorganyle wie z. B. n-Butyllithium, tert.-Butyllithium, Phenyllithium oder Alkalimetallamide wie Lithium-, Kalium- oder Natriumamid, Lithiumdiisopropylamid, aber auch Alkalimetallhexamethyldisilazide in Frage.

Wenn X- ein Halogenidanion ist, können auch Oxirane vorteilhaft als latente Basen eingesetzt werden (siehe Chem. Ber. 1974, 107, 2050).

Vorzugsweise werden für diese Wittig-Reaktion als Basen Lösungen von Alkalimetallalkoholaten im korrespondierenden Alkohol oder Oxirane, vor allem 1,2-Epoxybutan, ohne zusätzliches Solvens oder im Gemisch mit einem der oben genannten Lösungsmittel oder einem niederen Alkanol verwendet.

Die Menge an eingesetzter Base liegt in der Regel im Bereich von 0,8 bis 5 Mol, bevorzugt 1 bis 3 Mol pro Mol des eingesetzten Phosphoniumsalzes III.

Für die Wittig-Horner-Reaktion von II mit einer Verbindung der Formel III, worin (P) für einen Phosphonsäuredialkylesterrest steht, kommen ebenfalls die für diese Umsetzung typischen Bedingungen zur Anwendung. Vorzugsweise arbeitet man auch hier in einem der vorstehend genannten inerten organischen Lösungsmittel und setzt als Base bevorzugt die Lösung eines Alkalimetallalkoholats im korrespondierenden Alkanol ein. Es ist im Falle der Wittig-Horner-Reaktion aber auch möglich, die oben für die Wittig-Reaktion zusätzlich genannten Basen, mit Ausnahme der Oxirane, zu verwenden.

Der Schritt b) des erfindungsgemäßen Verfahrens ist fakultativ. Im Schritt b) wird der C₁₅-Baustein IV in einen um fünf Kohlenstoffatome verlängerten C₂₀-Baustein der Formel VI umgewandelt. Hierzu wird in der Verbindung der Formel IV zunächst die Acetalfunktion zur Aldehydfunktion hydrolysiert. Prinzipiell sind hier alle dem Fachmann bekannten Bedingungen zur vorzugsweise sauer katalysierten Acetalspaltungen geeignet, z. B. mit verdünnten Mineralsäuren wie Schwefelsäure. Es hat sich als besonders geeignet erwiesen, die Hydrolyse der Acetalfunktion mit Citronensäure zu katalysieren. Die Citronensäure wird zweckmäßigerweise in einer Menge von 5 bis 50 Mol-%, vorzugsweise 20 bis 30 Mol-%, bezogen auf die Verbindung der Formel IV eingesetzt. Die Hydrolyse erfolgt vorzugsweise in wässrigen Medien, insbesondere in einem Gemisch von Wasser mit einem wassermischbaren organischen Lösungsmittel, wie C₁-C₄-Alkanolen, z. B. Ethanol bei einer Temperatur von geeigneterweise 0 °C bis zum Siedepunkt des Lösungsmittels, vorzugsweise 25 bis 55 °C.

Der erhaltene Esteraldehyd wird dann mit einem Reagenz der Formel V 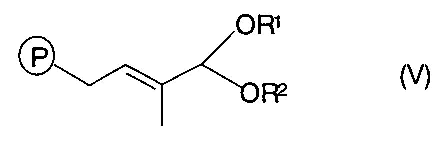 umgesetzt, worin R¹, R² und (P) die bereits angegebenen Bedeutungen haben. Die Umsetzung erfolgt unter Bedingungen, die für eine Wittig- bzw. Wittig-Horner-Reaktion typisch sind, wozu auf die vorstehenden Ausführungen verwiesen wird.

Verbindungen der Formel V sind an sich bekannt. Sie sind z. B. erhältlich aus β-Formyl-crotylacetat. Ihre Herstellung ist z. B. in Carotenoids, Vol. 2 "Synthesis", S. 115ff, Birkhäuser Verlag, 1996 beschrieben.

Bevorzugt setzt man im Schritt b) ein Reagenz der Formel Va ein, 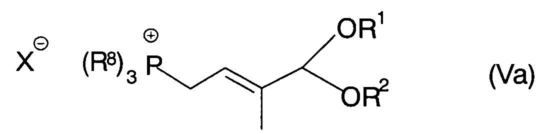 worin R¹, R², R⁸ und X- die bereits angegebene Bedeutung haben.

In den Schritten c) und d) des erfindungsgemäßen Verfahrens wird die Esterfunktion in der Verbindung der Formel IV oder VI in zwei Schritten zur Aldehydfunktion umgewandelt.

Die zweistufige Umwandlung hat sich als günstiger erwiesen als die direkte Umwandlung des Esters zum Aldehyd. Im Schritt c) wird die Esterfunktion zuerst zum Alkohol reduziert. Für diesen Schritt können prinzipiell alle dem Fachmann bekannten Reagenzien zur Reduktion von Estern zu Alkoholen, vorzugsweise Hydridreagenzien, eingesetzt werden, beispielsweise Alkalimetallbor- oder Alkalimetallaluminiumhydride.

In einer bevorzugten Ausführungsform von Verfahrensschritt c) verwendet man zur Reduktion der Esterfunktion eine Natrium-Aluminium-Hydridverbindung, besonders bevorzugt Natrium-dihydrobis-(2-methoxyethoxy)-aluminat. Die handelsübliche konzentrierte toluolische Lösung von Natrium-dihydrido-bis-(2-methoxyethoxy)-aluminat ("Vitride®") ist besonders vorteilhaft. Dieses Reagens ist nicht pyrophor, nicht empfindlich gegenüber Sauerstoff (GIT Fachz. Lab. 9/96, 914) und als Flüssigkeit in einem technischen Prozess wesentlich einfacher handhabbar als feste komplexe Hydride wie z. B. Lithiumaluminiumhydrid.

Die Reaktion wird vorzugsweise so durchgeführt, dass man den Ester der Formel IV oder VI in einem gegenüber Hydridreagenzien inerten Lösungsmittel wie aromatischen Kohlenwasserstoffen, z. B. Toluol, offenkettigen oder cyclischen Ethern, Glycolethern oder in einem Gemisch dieser Solventien vorlegt und das Reduktionsmittel im Temperaturbereich von -20 °C bis 30 °C, bevorzugt von -10 °C bis 10 °C, besonders bevorzugt von -5 °C bis 0 °C zudosiert.

In der Regel werden pro Äquivalent Ester mindestens zwei Äquivalente Hydrid eingesetzt, d. h. mindestens 0,5 Mol Lithiumaluminiumhydrid/mol Ester bzw. 1,0 Mol Vitride/mol Ester. Um einen vollständigen Umsatz zu erzielen, ist es jedoch vorteilhaft, einen Überschuss an Reduktionsmittel einzusetzen. Dieser Überschuss liegt im Bereich von 10 bis 50 Mol-%, vorzugsweise 20 bis 30 Mol-%.

In der bevorzugten Ausführungsform des erfindungsgemäßen Verfahrens wird der Ester der Formel IV oder VI mit der toluolischen Vitride-Lösung zum Alkohol VII reduziert. Nach wässriger Aufarbeitung erhält man in praktisch quantitativer Ausbeute ein Rohprodukt, das ohne Reinigung direkt in die Folgestufe d) eingesetzt werden kann.

Zur Oxidation der Verbindung der Formel VII zur Verbindung der Formel I im Schritt d) des erfindungsgemäßen Verfahrens kommen die dem Fachmann bekannten Oxidationsverfahren zur Überführung von Polyenalkoholen in Polyenaldehyde in Frage, die z. B. in der DE-A-3705785, DE-A-4440286, DE-A-4440287 sowie in der EP-A-0 718 283 beschrieben sind. Unter ökonomischen, ökologischen und verfahrenstechnischen Gesichtspunkten sind katalytische Methoden bevorzugt. Als Katalysatoren hierfür sind unter anderem Ruthenium-Verbindungen, wie Tetrapropylammonium-perruthenat, Tris(triphenylphosphin)ruthenium(II)-chlorid oder 1,5-Cyclooctadien-ruthenium(II)-chlorid in Mengen von 2 bis 4 Mol-% in Gegenwart einer wenigstens stöchiometrischen Menge an 4-Methyl-morpholin-N-oxid als Co-Oxidans einsetzbar (siehe J. Chem. Soc. Chem, Commun. 1987, 1625).

Vorzugsweise oxidiert man die Verbindung der Formel VII jedoch mit Sauerstoff in Gegenwart eines N-Oxylradikals und einer Kupfer(I)-Verbindung. Die N-Oxylradikale leiten sich üblicherweise von sekundären Aminen ab, worin alle Substituenten an den α-Kohlenstoffatomen zum Stickstoffatom von Wasserstoff verschieden sind. Geeignete N-Oxylradikale sind z. B. 2,2,6,6-Tetramethyl-piperidin-1-oxyl und 4-Hydroxy-2,2,6,6-tetramethyl-piperidin-1-oxyl. Als Kupfer(I)-Verbindung ist Kupfer(I)-chlorid bevorzugt. Die katalytische Oxidation von VII zu I erfolgt dabei bevorzugt mit einem Gemisch, das 2,2,6,6-Tetramethyl-piperidin-1-oxyl/Kupfer(I)chlorid/Dimethylformamid/Sauerstoff enthält oder mit einem Gemisch, das 4-Hydroxy-2,2,6,6-tetramethyl-piperidin-1-oxyl/Kupfer(I)chlorid/Dimethylformamid/Sauerstoff enthält, in Dimethylformamid als Solvens. Nähere Einzelheiten zur Oxidation finden sich u. a. in DE-A-3705785 und EP-A-0 718 283.

Die Erfindung betrifft außerdem Verbindungen der Formel 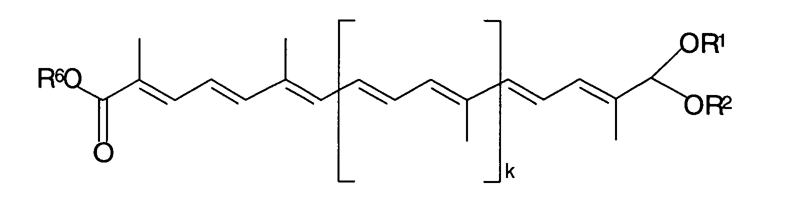 worin R¹, R², R⁶ und k die bereits angegebenen Bedeutungen haben, wobei für den Fall, dass R¹ und R² gemeinsam mit den Sauerstoffatomen, an die sie gebunden sind, und dem dazwischen liegenden Kohlenstoffatom für 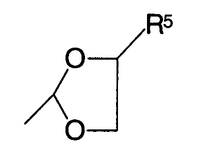 stehen, R⁵ nicht für Wasserstoff steht, wenn k für 0 und R⁶ für Methyl steht.

Bevorzugte Verbindungen weisen die Formel 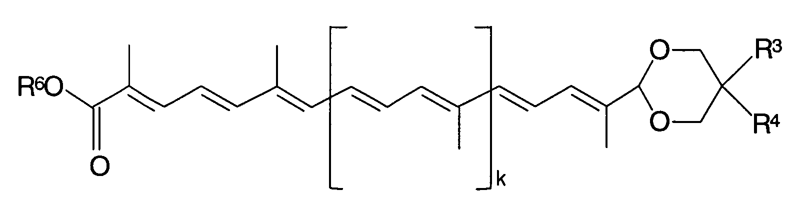 auf, worin R⁶ für C₁-C₄-Alkyl steht und R³ und R⁴ gleich sind und entweder für Wasserstoff oder Methyl stehen.

Die Erfindung betrifft außerdem Verbindungen der Formel VII 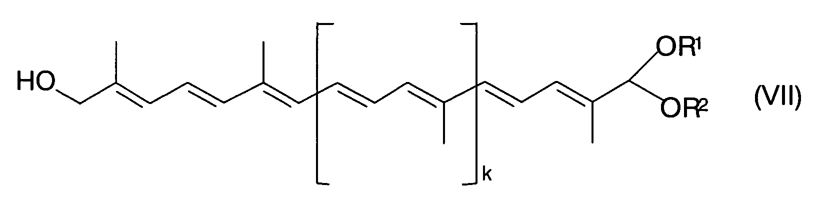 worin R¹, R² und k die bereits angegebenen Bedeutungen haben. Bevorzugte Verbindungen weisen die Formel 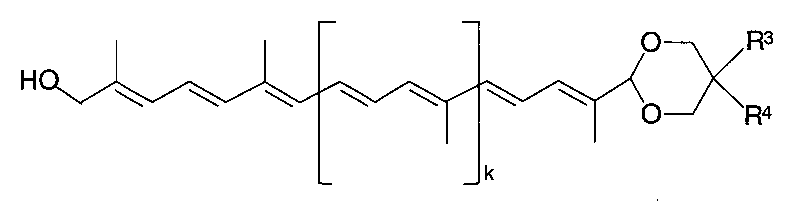 auf, worin R³ und R⁴ gleich sind und für Wasserstoff oder Methyl stehen.

Die Erfindung betrifft außerdem Verbindungen der Formel I 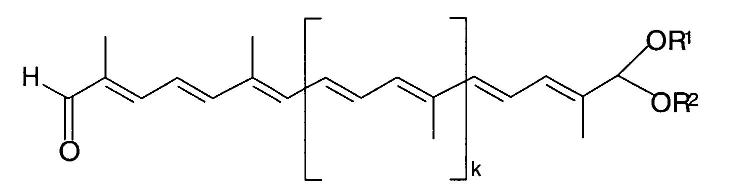 worin R¹, R² und k die bereits angegebene Bedeutung haben, wobei für den Fall, dass R¹ und R² gemeinsam mit den Sauerstoffatomen, an die sie gebunden sind, und dem dazwischen liegenden Kohlenstoffatom für 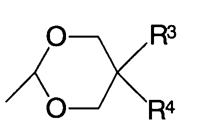 stehen, R³ und R⁴ nicht gleichzeitig für Methyl stehen.

Die Erfindung wird durch die folgenden Beispiele näher veranschaulicht.

### Beispiel 1: 11-(5,5-Dimethyl-1,3-dioxan-2-yl)-2,6-dimethyl-2,4,6,8,10-dodecapentaenal

### a) 11-(5,5-Dimethyl-1,3-dioxan-2-yl)-2,6-dimethyl-2,4,6,8,10-dodecapentaensäure-ethylester

62,6 g (0,25 Mol) 2,7-Dimethyl-2,4,6-octatrien-1,8-dial-mononeopentylacetal und 79,3 g (0,30 Mol) 4-(Diethylphosphono)-2-methyl-2-butensäure-ethylester wurden in 625 ml Dichlormethan vorgelegt. Bei 0 °C ließ man innerhalb von 60 Minuten 112,3 g einer 20%igen ethanolischen Lösung von Natriumethylat (0,33 Mol) zulaufen. Man rührte 1 Stunde bei 0 °C und anschließend 24 Stunden bei Raumtemperatur. Danach wurde der Ansatz mit 100 ml 10%iger wässriger Essigsäure und 250 ml halbkonzentrierter Kochsalzlösung versetzt. Die organische Phase wurde abgetrennt, je einmal mit 250 ml halbkonzentrierter Kochsalzlösung und Wasser gewaschen, über Natriumsulfat getrocknet und im Vakuum bei 60 °C eingeengt. Ausbeute: 101,4 g rotbraunes viskoses Öl.

Zur Charakterisierung wurde das Rohprodukt in einem Gemisch aus 600 ml Isobutanol und 5 ml Triethylamin in der Hitze gelöst. Man ließ auf 0 °C abkühlen, rührte 2 Stunden bei 0 °C und filtrierte das gebildete Kristallisat ab. Der Filterkuchen wurde mit kaltem Isobutanol gewaschen und im Stickstoffstrom getrocknet. Ausbeute an Kristallisat: 51,5 g (57,2 % d. Th.). Smp. 118,5 bis 119 °C. E¹₁ (CHCl₃): 2055 (370 nm), 1818 (390 nm).

Das Filtrat wurde im Vakuum bei 50 °C eingeengt. Man erhielt 52,8 g rotbraunes Öl.

### b) 11-(5,5-Dimethyl-1,3-dioxan-2-yl)-2,6-dimethyl-2,4,6,8,10-dodecapentaenol

27,05 g (75 mmol) des kristallinen Produkts aus Schritt a) wurden in 300 ml THF gelöst. Bei 0 °C ließ man innerhalb einer Stunde 23,8 g einer 70%igen toluolischen Lösung von Natriumdihydrobis-(2-methoxy-ethoxy)-aluminat und 300 ml Toluol zulaufen. Das Reaktionsgemisch wurde bei 0 °C mit 300 ml Hexan verdünnt. Bei 0 bis 5 °C ließ man 300 ml halbkonzentrierte Kochsalzlösung zulaufen. Die wässrige Phase wurde abgetrennt und zweimal mit je 300 ml eines Gemischs aus Toluol/n-Hexan (1:1 Vol/Vol) nachextrahiert. Die vereinigten organischen Phasen wurden einmal mit 300 ml halbkonzentrierter Kochsalzlösung gewaschen, über Natriumsulfat getrocknet und am Rotationsverdampfer eingeengt. Der Rückstand (28,8 g) wurde mittels Flash-Chromatographie an Kieselgel (Eluierungsmittel: Cyclohexan/Essigester 10:1) gereinigt. Man erhielt 14,5 g (61,4 % der d. Th.) der Titelverbindung. Zur Charakterisierung wurde eine Probe aus Diisopropylether kristallisiert. Man erhielt gelbe Kristalle mit Smp. 136 bis 137 °C. E¹₁ (CHCl₃): 1096 (333 nm), 2668 (350 nm), 2495 (339 nm).

### c) 11-(5,5-Dimethyl-1,3-dioxan-2-yl)-2,6-dimethyl-2,4,6,8,10-dodecapentaenal

3,17 g des Produkts aus Schritt b) wurden in 12,5 ml Dimethylformamid gelöst. Man gab 79,7 mg (0,5 mmol) 2,2,6,6-Tetramethylpiperidin-1-oxyl und 51,03 mg (0,5 mmol) Kupfer(I)-chlorid dazu. Dann begaste man 3 Stunden bei 20 bis 25 °C mit Sauerstoff. Man gab nochmals dieselbe Menge beider Katalysatoren zu und begaste eine weitere Stunde bei 20 bis 25 °C mit Sauerstoff. Das Reaktionsgemisch wurde mit je 50 ml halbkonzentrierter Kochsalzlösung und Essigester versetzt. Die wässrige Phase wurde abgetrennt und dreimal mit je 50 ml Essigester nachextrahiert. Die vereinigten organischen Phasen wurden mit 50 ml halbkonzentrierter Kochsalzlösung gewaschen, über Natriumsulfat getrocknet und am Rotationsverdampfer eingeengt. Der Rückstand (3,52 g) wurde durch Flash-Chromatographie an Kieselgel (Eluierungsmittel: Cyclohexan-Essigester 8:1) gereinigt. Man erhielt 2,33 g (73,7 % d. Th.) der Titelverbindung. Zur Charakterisierung wurde eine Probe aus Diisopropylether umkristallisiert. Smp. 139,5 bis 140 °C, E¹₁ (CHCl₃): 2052 (385 nm), 1969 (401 nm).

### Beispiel 2: 15-(5,5-Dimethyl-1,3-dioxan-2-yl)-2,6,11-trimethyl-2,4,6,8,10,12,14-hexadecaheptaenal (Crocetindial-mononeopentylglycolacetal)

### a) 11-(5,5-Dimethyl-1,3-dioxan-2-yl)-2,6-dimethyl-2,4,6,8,10-dodecapentaensäure-ethylester

Die Verbindung wurde hergestellt, wie im Beispiel 1, Schritt a) beschrieben. Der Eindampfrückstand wurde ohne weitere Reinigung im nachfolgenden Schritt b) verwendet.

### b) 2,6,11-Trimethyl-12-oxo-2,4,6,8,10-dodecapentaensäureethylester

52,8 g des Eindampfrückstandes aus Schritt a) wurden in 440 ml Ethanol gelöst. Man gab eine Lösung von 6,16 g (29,3 mmol) Citronensäure-monohydrat in 100 ml Wasser dazu und erhitzte 1 Stunde auf 50 °C. Dann kühlte man auf 0 °C ab und rührte 24 Stunden bei 0 °C nach. Das Kristallisat wurde abgesaugt, zweimal mit je 50 ml eines Ethanol-Wasser-Gemischs (8:2 Vol./Vol.) und einmal mit 50 ml heißem Wasser gewaschen und anschließend im Vakuumtrockenschrank bei 50 °C zur Gewichtssubstanz getrocknet. Ausbeute 10,1 g Smp. 120-121 °C.

### c) 15-(5,5-Dimethyl-1,3-dioxan-2-yl)-2,6,11-trimethyl-2,4,6,8,10,12,14-hexadecaheptaensäure-ethylester.

13,7 g (50 mmol) des Produkts aus Schritt b) wurden in 125 ml Dichlormethan gelöst. Man gab 25,7 g (55 mmol) 3-(5,5-Dimethyl-1,3-dioxan-2-yl)but-2-enyl-triphenylphosphoniumchlorid zu. Bei 0 °C gab man innerhalb von 1 Stunde 20,4 g einer 20%igen ethanolischen Lösung von Natriumethylat zu (60 mmol). Man rührte 1 Stunde bei 0 °C nach und versetzte dann mit 50 ml halbkonzentrierter Kochsalzlösung. Die organische Phase wurde abgetrennt, einmal mit 50 ml halbkonzentrierter Kochsalzlösung gewaschen, über Natriumsulphat getrocknet und am Rotationsverdampfer bei 20 °C bis 20 mbar eingeengt. Der Rückstand (38,2 g) wurde mittels Flash-Chromatographie an Kieselgel (Eluierungsmittel: Cyclohexan/Methyl-t-butylether 4:1) aufgereinigt. Man erhielt 19,9 g (93,4 % der Theorie) der Titelverbindung. Zur Charakterisierung wurde das Produkt in der Hitze in 100 ml Essigester gelöst. In der Hitze gab man 50 ml Diisopropylether dazu, kühlte auf 0 °C ab und rührte 1 Stunde weiter. Das gebildete Kristallisat wurde abfiltriert, mit kaltem Essigester-Diisopropylether-Gemisch (2:1 Vol./Vol.) gewaschen und im Stickstoffstrom getrocknet. Ausbeute: 8,9 g rote Kristalle. Smp. 157,5 bis 158 °C. E¹₁ (CHCl₃):2495 (423 nm), 2243 (448 nm).

### d) 15-(5,5-Dimethyl-1,3-dioxan-2-yl)-2,6,11-dimethyl-2,4,6,8,10,12,14-hexadecaheptaenol

42,3 g des Produkts aus Schritt c) wurden in 400 ml Tetrahydrofuran gelöst. Bei 0 °C ließ man innerhalb einer Stunde 31,8 g einer 70%igen toluolischen Lösung von Natrium-dihydrido-bis(2-methoxyethoxy)aluminat und 400 ml Toluol zulaufen und rührte 1 Stunde bei 0 °C nach. Dann verdünnte man das Reaktionsgemisch mit 400 ml n-Hexan und ließ innerhalb von 15 Minuten 400 ml halbkonzentrierte Kochsalzlösung zulaufen. Die wässrige Phase wurde zweimal mit je 400 ml eines 1:1-Gemisches (Vol./Vol.) von Toluol/Hexan nachextrahiert. Die vereinigten organischen Phasen wurden über Natriumsulfat getrocknet und am Rotationsverdampfer bei 50 °C bis 20 mbar eingeengt. Der Rückstand wurde mittels Flash-Chromatographie an Kieselgel (Eluierungsmittel: Cyclohexan/Essigester 10:1) gereinigt. Man erhält 20,12 g (55,2 % d. Th.) der Titelverbindung. Das Produkt wurde in dieser Form im nachfolgenden Schritt e) eingesetzt. E¹₁ (CHCl₃) 1579 (383 nm), 2668 (405 nm), 2919 (3430 nm).

### e) Crocetindial-mononeopentylglykolacetal

3,09 g des Produkts aus Schritt d) wurden in 11 ml Dimethylformamid gelöst. Man gab 64 mg (0,4 mmol) 2,2,6,6-Tetramethyl-piperidin-1-oxyl und 41 mg (0,4 mmol) Kupfer(I)chlorid zu. Dann begaste man 3 Stunden bei 20 bis 25 °C mit Sauerstoff. Man gab nochmals dieselbe Menge beider Katalysatoren zu und begaste eine weitere Stunde mit Sauerstoff. Dann wurde der Ansatz mit 40 ml halbkonzentrierter Kochsalzlösung und 40 ml Essigester versetzt. Die wässrige Phase wurde abgetrennt und mit 40 ml Essigester nachextrahiert. Die vereinigten organischen Phasen wurden über Natriumsulfat getrocknet und am Rotationsverdampfer eingeengt. Der Rückstand wurde durch Flash-Chromatographie an Kieselgel (Eluierungsmittel Cyclohexan/Essigester 10:1) gereinigt. Man erhielt 1,3 g (43 % d. Th.) Crocetindialdehyd-mononeopentylglycolacetal. Zur Charakterisierung wurde eine Probe aus Essigester/Diisopropylether umkristallisiert. Smp. 183,5 bis 184 °C. E¹₁ (CHCl₃) 2434 (438 nm).

## Patentansprüche

1. Verfahren zur Herstellung von Verbindungen der Formel I 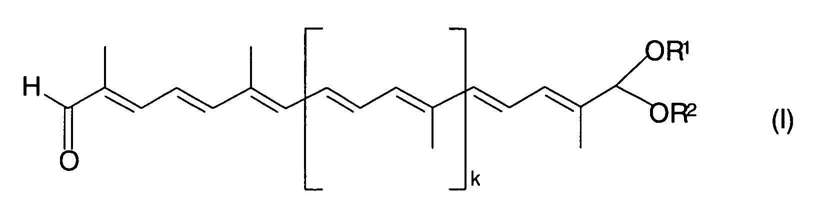 bei dem man
a) eine Verbindung der Formel II 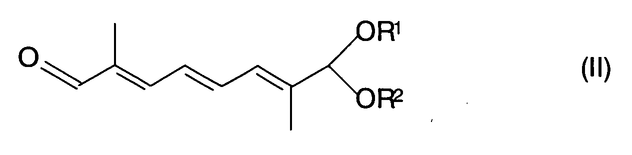 durch Umsetzung mit einem Reagenz der Formel III 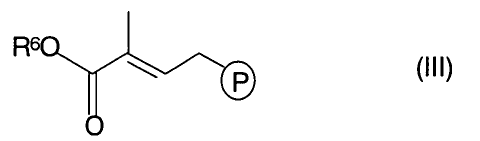 in eine Verbindung der Formel IV umwandelt, 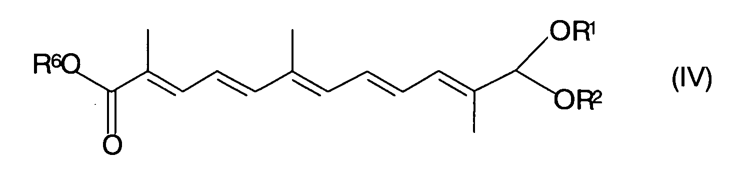
b) gegebenenfalls die Verbindung der Formel IV durch Hydrolyse der Acetalfunktion und Umsetzung mit einem Reagenz der Formel V 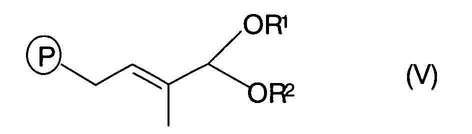 in eine Verbindung der Formel VI umwandelt 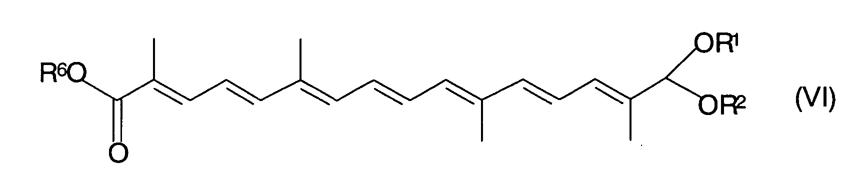
c) die Verbindung der Formel IV oder VI zu einer Verbindung der Formel VII reduziert 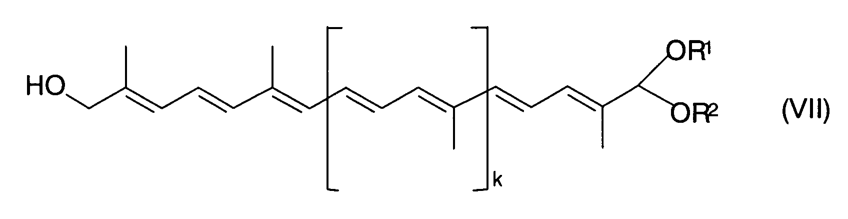
d) die Verbindung der Formel VII zur Verbindung der Formel I oxidiert,
worin R¹ und R² unabhängig voneinander für C₁-C₈-Alkyl stehen oder gemeinsam mit den Sauerstoffatomen, an die sie gebunden sind, und dem dazwischen liegenden Kohlenstoffatom für 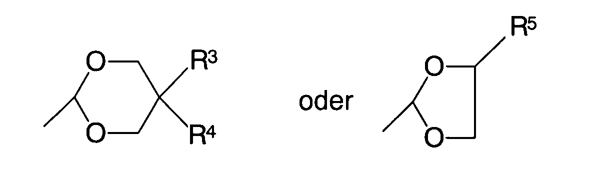 stehen, worin R³, R⁴ und R⁵ unabhängig voneinander für Wasserstoff oder C₁-C₄-Alkyl stehen,
R⁶ für C₁-C₈-Alkyl steht,
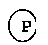 für einen Triarylphosphonium- oder Phosphonsäuredialkylesterrest steht, und
k für 0 oder 1 steht.

2. Verfahren nach Anspruch 1, bei dem man in Schritt a) eine Verbindung der Formel IIa einsetzt 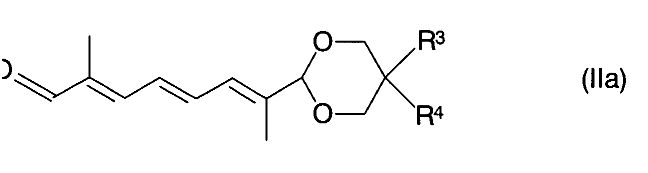 worin R³ und R⁴ gleich sind und für Wasserstoff oder Methyl stehen.

3. Verfahren nach Anspruch 1 oder 2, bei dem man in Schritt a) ein Reagenz der Formel IIIa einsetzt 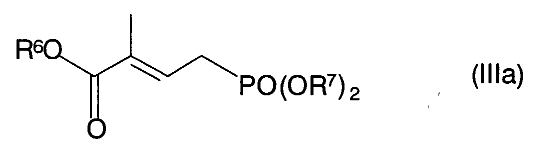 worin R⁶ die in Anspruch 1 angegebene Bedeutung hat und R⁷ für C₁-C₃-Alkyl steht.

4. Verfahren nach einem der vorhergehenden Ansprüche, bei dem man in Schritt b) die Hydrolyse der Acetalfunktion mit Citronensäure katalysiert.

5. Verfahren nach einem der vorhergehenden Ansprüche, bei dem man in Schritt b) ein Reagenz der Formel Va einsetzt 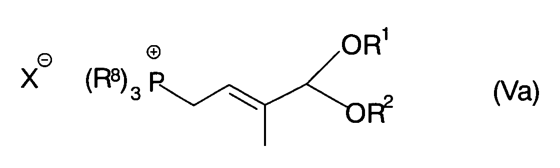 worin R¹ und R² die in Anspruch 1 angegebene Bedeutung haben und R⁸ für Aryl und X- für ein Anionenäquivalent einer anorganischen oder organischen Säure steht.

6. Verfahren nach einem der vorhergehenden Ansprüche, bei dem man in Schritt c) eine Natrium-Aluminium-Hydridverbindung als Reduktionsmittel verwendet.

7. Verfahren nach einem der vorhergehenden Ansprüche, bei dem man in Schritt d) die Verbindung der Formel VII mit Sauerstoff in Gegenwart eines N-Oxylradikals und einer Kupfer(I)-Verbindung oxidiert.

8. Verbindung der Formel 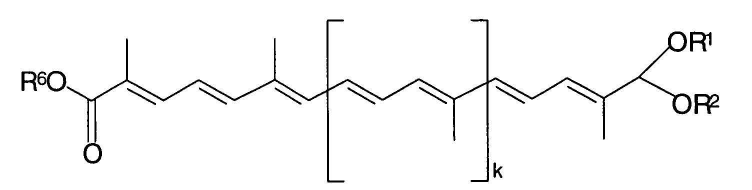 worin R¹ und R² unabhängig voneinander für C₁-C₈-Alkyl stehen oder gemeinsam mit den Sauerstoffatomen, an die sie gebunden sind, und dem dazwischen liegenden Kohlenstoffatom für 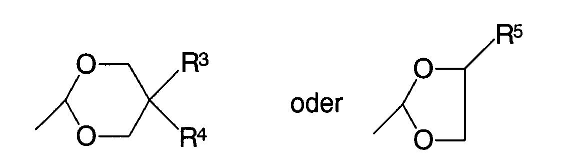 stehen, worin R³, R⁴ und R⁵ unabhängig voneinander für Wasserstoff oder C₁-C₄-Alkyl stehen,
R⁶ für C₁-C₈-Alkyl steht, und
k für 0 oder 1 steht,
wobei R⁵ nicht für Wasserstoff steht, wenn k für 0 und R⁶ für Methyl steht.

9. Verbindung der Formel 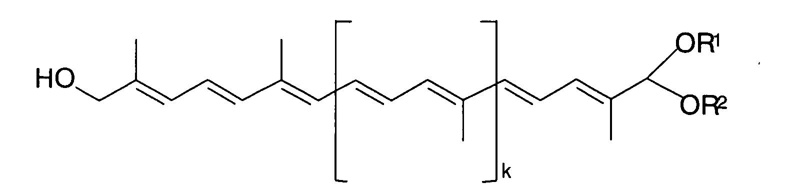 worin R¹ und R² unabhängig voneinander für C₁-C₈-Alkyl stehen oder gemeinsam mit den Sauerstoffatomen, an die sie gebunden sind, und dem dazwischen liegenden Kohlenstoffatom für 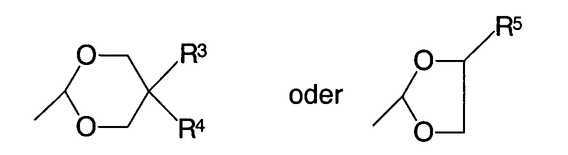 stehen, worin R³, R⁴ und R⁵ unabhängig voneinander für Wasserstoff oder C₁-C₄-Alkyl stehen, und
k für 0 oder 1 steht.

10. Verbindung der Formel 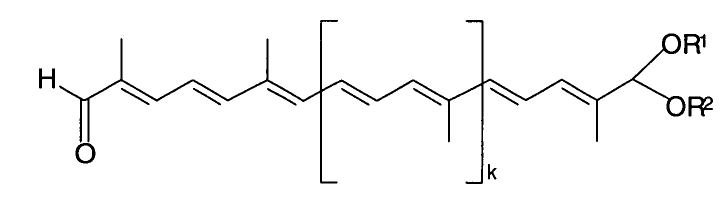 worin R¹ und R² unabhängig voneinander für C₁-C₈-Alkyl stehen oder gemeinsam mit den Sauerstoffatomen, an die sie gebunden sind, und dem dazwischen liegenden Kohlenstoffatom für 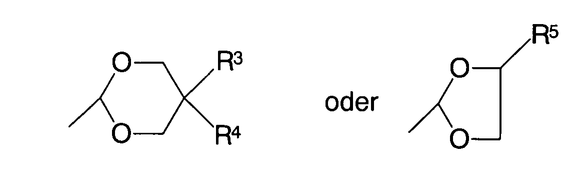 stehen, worin R³, R⁴ und R⁵ unabhängig voneinander für Wasserstoff oder C₁-C₄-Alkyl stehen, und
k für 0 oder 1 steht,
wobei R³ und R⁴ nicht gleichzeitig für Methyl stehen.
